# EUROPEAN PATENT SPECIFICATION

(11) **EP 3 866 936 B1**
(45) Date of publication and mention of the grant of the patent: **06.03.2024**
(21) Application number: 19778551.2
(22) Date of filing: 03.10.2019
(51) Int. Cl.: A61Q 19/00, A61K 8/891, A61K 8/73, A61K 8/81, A61K 8/25, A61K 8/92

(54) **COSMETIC COMPOSITION WITH AN IMPROVED PICKUP**
KOSMETISCHE ZUSAMMENSETZUNG MIT VERBESSERTER ABHOLUNG
COMPOSITION COSMÉTIQUE PRÉSENTANT UNE MEILLEURE PRISE

(30) Priority: 15.10.2018 WO PCT/CN2018/110192; 20.11.2018 EP 18207253
(43) Date of publication of application: 25.08.2021
(73) Proprietor: Unilever IP Holdings B.V., 3013 AL Rotterdam (NL); UNILEVER GLOBAL IP LIMITED, Wirral Merseyside CH62 4ZD (GB)
(72) Inventor: HU, Xiaobo, Shanghai, Linkong Economic Development Zone 200335 (CN); KAWADE, Jayshree, Mumbai 400 099 Chakala, Andheri (E) (IN); KINI, Mridula, Mumbai 400 099 Chakala, Andheri (E) (IN); MENG, Sheng, Shanghai, Linkong Economic Development Zone 200335 (CN); ZHANG, Qian, Shanghai, Linkong Economic Development Zone 200335 (CN)
(74) Representative: James, Helen Sarah
(86) International application number: PCT/EP2019/076794
(87) International publication number: WO 2020/078728

(56) References cited:
- JP-A- 2015 027 953
- US-A1- 2007 059 262
- US-A1- 2015 335 569

## Description

### Field of the invention

The present invention relates to a cosmetic composition.

### Background of the invention

People often try to take care of themselves and of their body surfaces e.g. skin, scalp including hairs, axilla and oral cavity, with a desire of enjoying a healthy lifestyle. Some of the benefits people tend to have desire for include healthy and infection-free skin, even skin tone, adequate moisturization and protection from ultraviolet rays contained in sunlight.

Skin is the outermost protective covering of living beings and is the largest organ in the body. It acts as a barrier and protects the body from external factors e.g. dust, dirt, pollution and ultraviolet radiation contained in sunlight. Skin also helps prevent entry of harmful or potentially harmful microbes e.g. bacteria, fungi and viruses, from entering the body thereby preventing infection and/or other ill effects that may be caused. However, being the outermost covering, skin is always exposed to one or more factors mentioned earlier. As a result, skin is susceptible to developing one or more conditions e.g. dryness, wrinkles, loose/saggy skin, age spots, blotchy skin, melasma, freckle and increased pigmentation, which may lead to less preferred uneven skin tone.

One of the ways to reduce occurrence of such conditions is to avoid exposure to factors causing such conditions. However, in many instances, avoiding exposure to factors e.g. sunlight, is difficult and at times; unavoidable. For reasons like these, consumers tend to rely on cosmetic compositions that, when applied on a surface of the human body e.g. skin, provide benefits such as moisturizing, anti-aging, skin lightening.

WO2014101702 (Unilever) discloses a cosmetic composition comprising film-forming polymer having a contact angle of at least 85°, wax, optical particle and at least 20% water by weight of the composition. The compositions disclosed therein, provide improved wash-off resistance, abrasion resistance and/or long-lasting deposition of beneficial agent.

Cosmetic compositions are available in many different forms e.g. a lotion, a cream and powders. Depending upon the product form, consumer tend to either overturn a container and/or squeeze to dispense desired amount of the composition on their hands before applying it to their body surface e.g. skin. In addition to using their hands as an applicator; consumers also tend to make use of applicators like a sponge or a brush, to take out desired amount of the composition. For example, while applying a cream contained in a container e.g. wide mouth jar, consumers often take out desired amount of a composition simply by using hands or by using a brush. Therefore, consumers tend to prefer cosmetic compositions that have an appropriate pickup, i.e. ease with which desired amount of a composition may be taken out from its container.

Pickup is one of the sensory properties in addition to fragrance, skin-feel and appearance, that are associated with cosmetic compositions. In general, in the field of cosmetic compositions, sensory properties play a key role in influencing consumers' preferences, likes and dislikes; and consumers often tend to associate good sensory properties with optimum functionality of cosmetic compositions.

Consumers tend to dislike compositions with inappropriate pickup as it forces them to keep repeating the process of taking out a composition from its container until a desired amount of the composition is taken out. At times, this leads to consumers applying the composition in excess amount than required. Additionally, cosmetic compositions with inappropriate pickup, tend to get exposed to microbes that may be present on hands of consumers and/or that present on sponges and brushes, more number of times as compared to cosmetic compositions that have appropriate pickup.

JP2015027953 A (Adeka Corp) discloses an eye shadow composition having a urethane polymer obtained by the reaction of a specific monohydroxy compound, a specific polyethyleneglycol, a specific monoglycerylether compound, and a specific isocyanate compound. The eye shadow contains MQ resin, stearyl alcohol, 0.5 wt% behenyl alcohol, 13 wt% mica, 1 wt% bentonite and water.

US2015335569 A (Unilever) discloses a cosmetic composition comprising film-forming polymer having a contact angle of at least 85°, wax, optical particle, and at least 20 wt% water.

Need therefore exists to provide cosmetic compositions that have improved pickup. It is therefore an objective of the present invention to provide cosmetic compositions that exhibit improved pickup.

Therefore, it is an objective of the present invention to provide cosmetic compositions that exhibit improved pickup.

It is another objective of the present invention to provide a cosmetic composition that exhibits improved pickup whilst providing wash-off resistance, abrasion resistance and/or long-lasting deposition of an active ingredient e.g. optical particles that provide instant glow, whitening and improved coverage to the skin.

It has now been found that a cosmetic composition comprising film forming polymer that has contact angle of at least 85° where contact angle means the angle at which a water/vapor interface meets a solid surface at a temperature of 25°C, in combination with certain amount of wax, inorganic structurant and certain amount of water and further comprising skin lightening agent, provides improved pickup. The composition also provides wash-off resistance, abrasion resistance and/or long-lasting deposition of the skin lightening agent and/or optical particles.

### Summary of the invention

In a first aspect, the present invention relates to a cosmetic composition comprising:
a) from 0.01 to 20 wt% film-forming polymer having a contact angle of at least 85° where contact angle means the angle at which a water/vapor interface meets a solid surface at a temperature of 25°C which is measured with a goniometer or other water droplet shape analysis systems with water droplet of 5 µL and at 25°C,
b) from 0.01 to 20 wt% wax,
c) from 11 to 25 wt% inorganic structurant selected from the group consisting of talc, silica, mica, alumina, clays and mixtures thereof; and
d) from 5 to 50 wt% water, wherein the composition further comprises skin lightening agent selected from niacinamide, vitamin B6, 12-hydroxystearic acid, glutathione precursors, galardin, 4-alkyl substituted resorcinol and mixtures thereof, and wherein the wax is selected from a group consisting of beeswax,

rice bran wax, montan wax, spermaceti wax, carnauba wax, candelilla wax,
sugarcane wax, insect wax, hydrocarbon wax and mixtures thereof,
wherein the composition further comprises from 10 to 40 wt% emollient selected from the group consisting of mineral oil, petroleum jelly, isoparaffins, polyalphaolefins, isohexadecane and mixtures thereof.

### Detailed description of the invention

Any feature of one aspect of the present invention may be utilized in any other aspect of the invention. The word "comprising" is intended to mean "including" but not necessarily "consisting of" or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "x to y" are understood to include x and y. When for a specific feature multiple preferred ranges are described in the format "x to y", it is understood that all ranges combining the different endpoints are also contemplated. Unless specified otherwise, amounts as used herein are expressed in percentage by weight based on total weight of the composition and is abbreviated as "wt%". The use of any and all examples or exemplary language e.g. "such as" provided herein is intended merely to better illuminate the invention and does not in any way limit the scope of the invention otherwise claimed.

The present invention relates to a cosmetic composition comprising a film-forming polymer having a contact angle of at least 85°, wax, inorganic structurant and water. The composition is as defined in the appended set of claims.

### Film-forming polymer

The cosmetic composition according to the present invention (the composition) comprises a film-forming polymer having a contact angle of at least 85° where contact angle means the angle at which a water/vapor interface meets a solid surface at a temperature of 25°C. Such an angle is measured with a goniometer or other water droplet shape analysis systems with water droplet of 5 µL and at 25°C.

The film-forming polymer may for example comprise silicone resin, chitosan, or a mixture thereof. More preferably, the film-forming polymer comprises silicone resin and most preferably the film-forming polymer is silicone resin. The silicone resin is typically described by the following siloxy monomeric units:

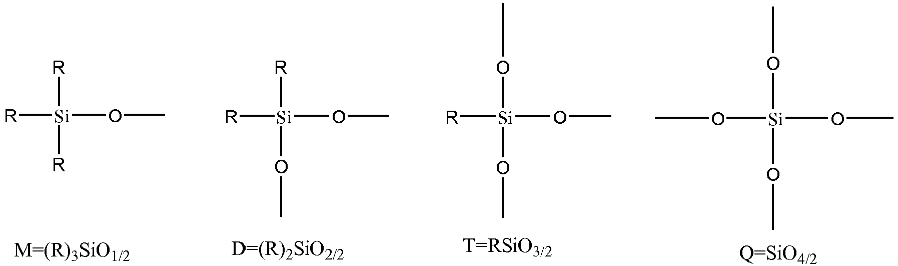

The R group may be selected from saturated or unsaturated hydrocarbon groups. Preferably, the silicone resin of the present invention may be selected from siloxysilicate, silsesquioxane, or a mixture thereof. More preferably, the silicone resin comprises M unit, Q unit, T unit or combination thereof. Even more preferably, the silicone resin comprises MQ silicone resin, T silicone resin, or a mixture thereof.

The silicone resin preferably comprises MQ silicone resin having the formula of [(R₁)₃-Si-O_{1/2]a}-(Si-O_{4/2})_{b}, wherein R₁ is mutually identical or different, selected from saturated hydrocarbon groups. R₁ is preferably selected from C₁ to C₆ alkyl, and more preferably each R₁ is methyl group. Thus, the more preferred MQ silicone resin is trimethylsiloxysilicate. Preferably, a and b independently have values ranging from 10 to 1000, and more preferably from 30 to 200.

The silicone resin preferably comprises T silicone resin having the formula of [R₂-Si-O_{3/2}]ₓ, wherein R₂ is selected from saturated hydrocarbon groups. R₂ is preferably selected from C₁ to C₆ alkyl, more preferably selected from methyl, ethyl, propyl, butyl, and most preferably propyl. The most preferred T silicone resin is polypropyl silsesquioxane. Preferably, x is less than 2000, more preferably less than 500, but preferably greater than 10, and more preferably greater than 50.

Most preferably, the silicone resin comprises a blend of MQ silicone resin and T silicone resin. The weight ratio of the MQ silicone resin to the T silicone resin is preferably from 1:20 to 20:1 in order to achieve better film-forming performance. More preferably, the weight ratio of the MQ silicone resin to the T silicone resin is from 1:10 to 10:1, even more preferably from 1:5 to 5:1.

Exemplary silicone resin suitable for the present invention includes Dow Corning^{™} MQ-1640 Flake Resin, a blend of MQ and T Propyl resins, Dow Corning^{™} MQ-1600 Solid Resin, a 100% active MQ resin, Dow Corning^{™} 670 Fluid, Cyclopentasiloxane (and) Polypropylsilsesquioxane supplied by Dow Corning.

The film-forming polymer is present in the composition in an amount from 0.01 to 20 wt%, preferably from 0.2 to 10 wt%, more preferably from 0.5 to 7 wt%, even more preferably from 1 to 4 wt% and most preferably from 2 to 4 wt%.

For better performance of wash-off resistance, the film-forming polymer preferably has a contact angle of at least 90°, more preferably from 95° to 160°, most preferably from 100° to 120°.

### Wax

The composition comprises wax. The wax may be natural wax and/or synthetic wax. Such waxes are often selected from hydrocarbon waxes and ester waxes out of which hydrocarbon wax are preferred. The wax is selected from beeswax, rice bran wax, montan wax, spermaceti wax, carnauba wax, candelilla wax, sugarcane wax, insect wax, polyethylene wax or a mixture thereof. More preferably, the wax comprises hydrocarbon wax (commercially available as Performalene^{®} 400 from New Phase Technologies; Jojoba wax , sunflower wax, plant based waxes) or mixtures thereof.

The wax is present in the composition in an amount from 0.01 to 20 wt%, preferably from 0.1 to 18 wt%, more preferably from 0.2 to 16 wt% and even more preferably from 0.5 to 16 wt%, further more preferably from 1 to 16 wt%, still more preferably from 5 to 16 wt%.

Without wishing to be bound to any theory or explanation, it is believed that the wax was embedded into the network of film-forming polymer layer to form a compact film.

Such film has stronger binding force to the substrates and improved the performance of wash-off resistance. Therefore, to form a stronger film and/or be better compatible with the film-forming polymer, the wax preferably has a melting point from 40 to 200 °C, more preferably from 50 to 120 °C, even more preferably from 60 to 100°C.

### Inorganic structurant

The composition comprises an inorganic structurant selected from the group consisting of talc, silica, mica, alumina, clays and mixtures thereof. Examples of clays include kaolin, attapulgite, bentonites, hectorites and attapulgite.

The composition comprises from 11 to 25 wt%, preferably from 13 to 20 wt%, more preferably from 13 to 19 wt% and even more preferably from 13 to 18 wt% inorganic structurant.

The inorganic structurant present in amounts as per the present invention, improves the structure of the composition thereby resulting in improved pickup of the composition.

### Water

The composition comprises water. Water comprised in the composition acts as a solvent and provides hydrating effect.

The composition comprises from 5 to 60 wt%, preferably from 10 to 50 wt%, more preferably from 15 to 45 wt%, even more preferably from 20 to 45 wt% water.

### Optical Particle

Preferably, the composition further comprises optical particle. Without being bound to any theory or explanation, it is believed that optical particles when present, would be embedded into the film by film-forming polymer and wax. Therefore, the optical particles are able to resist water and/or friction and deliver the long-lasting opacity to the skin, when incorporated.

Examples of such optical particles are those comprising bismuth oxy-chloride, boron nitride, barium sulfate, mica, silica, titanium dioxide, zirconium oxide, iron oxide, aluminium oxide, zinc oxide or combinations thereof. More preferred particles are particles comprising titanium dioxide, zinc oxide, zirconium oxide, iron oxide or a combination thereof. Even more preferred particles are particles comprising zinc oxide, zirconium oxide, titanium dioxide or a combination thereof as these materials have especially high refractive index. Most preferred is titanium dioxide.

The optical particles are typically particles of high refractive index materials. For example, the optical particles may have a refractive index of greater than 1.3, more preferably greater than 1.7 and most preferably from 2.0 to 2.7.

For sake of good compatibility with the film-forming polymer and/or wax, the optical particle is preferably hydrophobic. More preferably, the optical particle is preferably hydrophobically modified. Even more preferably the optical particle is modified by hydrophobic material selected from fatty acid, silicone oil, wax, and a mixture thereof. The fatty acid preferably comprises oleic acid, stearic acid, or a mixture thereof.

The size of optical particle is preferably from 2 nm to 5 microns, more preferably from 5 nm to 1 micron, even more preferably from 10 nm to 500 nm. Particle size as used herein refers to the diameter of particles in an unaggregated state. Diameter means the largest measurable distance on a particle in the event a well-defined sphere is not generated. The diameter may be measured for example by scanning electron microscopy (SEM) by averaging the value of at least ten particles.

Preferably the composition comprises optical particles in an amount of from 0.001 to 10 wt%, more preferably 0.01 to 7 wt%, more preferably still 0.05 to 5 wt% and most preferably 0.1 to 2 wt%.

The weight ratio of the film-forming polymer to the optical particle is preferably in the range of from 1:10 to 50:1, more preferably from 1:3 to 10:1, and most preferably from 1:1 to 5:1. The weight ratio of the wax to the optical particle is preferably in the range of from 1:40 to 20:1, more preferably from 1:20 to 10:1, and most preferably from 1:10 to 5:1.

### Skin Lightening agent

The composition of the invention further comprises skin lightening agent selected from niacinamide, vitamin B6, 12-hydroxystearic acid, glutathione precursors, galardin, 4-alkyl substituted resorcinol and mixtures thereof. Preferred 4-alkyl substituted resorcinol include phenylethyl resorcinol, butyl resorcinol and hexyl resorcinol. Other preferred skin lightening agents include galardin, adapalene, aloe extract, ammonium lactate, arbutin, azelaic acid, butyl hydroxy anisole, butyl hydroxy toluene, citrate esters, deoxyarbutin, 1,3-diphenyl propane derivatives, 2,5-dihydroxybenzoic acid and its derivatives, 2-(4-acetoxyphenyl)-1,3-dithiane, 2-(4-hydroxyphenyl)-1,3-dithiane, ellagic acid, gluco pyranosyl-1-ascorbate, gluconic acid, glycolic acid, green tea extract, 4-Hydroxy-5-methyl-3[2H]-furanone, 4-hydroxyanisole and its derivatives, 4-hydroxybenzoic acid derivatives, hydroxycaprylic acid, inositol ascorbate, lactic acid, lemon extract, linoleic acid, magnesium ascorbyl phosphate, 5-octanoyl salicylic acid, salicylic acid, 3,4,5-trihydroxybenzyl derivatives, acetylglucosamine, pitera extract, symwhite, calcium pantothenate (Melano-block), seppiwhite, soybean extract (bowman birk inhibitor), 12-hydroxystearic acid and mixtures thereof. When used in the composition, 12-hydroxystearic acid is used as a skin lightening agent and not as a fatty acid.

The alkyl group in 4-alkyl substituted resorcinol can be straight chain alkyl or branched chain alkyl. For example, the alkyl group can be straight chain alkyl as in the case of 4-propyl resorcinol or it can be a branched chain alkyl like as in the case of 4-isopropyl resorcinol. Examples of 4-alkyl substituted resorcinol include 4-methyl resorcinol, 4-ethyl resorcinol (ER), 4-propyl resorcinol, IPR, 4-butyl resorcinol, 4-pentyl resorcinol, 4-hexyl resorcinol (HR), 4-heptyl resorcinol, 4-octyl resorcinol and mixtures thereof. Preferred 4-alkyl substituted resorcinol are ER, HR and mixtures thereof.

The one or more skin lightening agents are preferably in an amount from 0.001 to 15 wt%, more preferably from 0.01 to 10 wt%, even more preferably from 0.1 to 5 wt%, further more preferably from 0.25 to 5 wt%, in the composition.

### Emollients

The composition further comprises from 10 to 40 wt% emollient selected from the group consisting of mineral oil, petroleum jelly, isoparaffins, polyalphaolefins, isohexadecane and mixtures thereof.

Emollients generally provide moisturizing and soft-feel to the skin.

Hydrocarbons emollient is selected from petroleum jelly, mineral oil, C₁₁-C₁₃ isoparaffins, polyalphaolefins, and especially isohexadecane (commercially available as Permethyl 101A from Presperse Inc.).

The composition comprises from 10 to 40 wt%, preferably from 12 to 38 wt%, more preferably from 15 to 35 wt%, even more preferably from 17 to 30 wt%, further more preferably from 17 to 28 wt%, still more preferably from 17 to 24 wt% emollient. Preferably, the composition further comprises humectants, preferably of polyhydric alcohol type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, glycerol, ethoxylated glycerol, propoxylated glycerol and mixtures thereof.

Preferably, the humectant selected is glycerol (also known as glycerin). Preferably, the composition comprises from 1 to 50 wt%, more preferably from 5 to 35%, even more preferably from 15 to 30 wt% humectant.

### Sunscreens

### UVA Sunscreen

Preferably, the composition further comprises UVA organic sunscreens that absorbs UVA radiations and prevent them from reaching a surface e.g. skin of a user.

Examples of UVA organic sunscreens that may be used in the composition include dibenzoyl methane compound, bisdisulizole disodium (commercially available as Neo Heliopan^{®} AP), diethylamino hydroxy benzoyl hexyl benzoate (commercially available as Uvinul^{®} A Plus), Ecamsule (commercially available as Mexoryl SX) and Methyl anthranilate and also the class of water soluble sunscreens e. g. benzophenone -4, Bisdisulizole disodium and disodium phenyl do benzimidazole tetrasulphonate

Preferably, UVA organic sunscreen that may be used as UVA sunscreen in the composition is selected from a dibenzoylmethane compound.

Examples of sunscreen of dibenzoyl methane compound that may be used as UVA organic sunscreen in the composition include 4-tert-butyl-4'-methoxydibenzoylmethane (BMDM; commercially available as Parsol^{®} 1789 or Avobenzone), 2-methyldibenzoylmethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane or 2,6-dimethyl-4-tert-butyl-4'-methoxydibenzoylmethane.

Most preferably, dibenzoylmethane compound that may be used as UVA organic sunscreen is BMDM.

When incorporated in the composition, UVA organic sunscreens may preferably be incorporated from 0.1 to 10 wt%, more preferably from 0.5 to 7 wt%, even more preferably from 1 to 5 wt%, further more preferably from 1 to 3.5 wt%, yet more preferably 1 to 3 wt%, still more preferably 1 to 2.5 wt% in the composition.

### UVB organic Sunscreen

Preferably, the composition further comprises UVB organic sunscreens that absorbs UVB radiations and prevent them from reaching a surface e.g. skin of a user.

Examples of UVB organic sunscreens that may be used in the composition include compounds from the class of cinnamic acid, salicylic acid, diphenyl acrylic acid and derivatives thereof. Examples of such compounds include 2-ethylhexyl salicylate (commercially available as Octisalate^{™}), 3,3,5-Trimethylcyclohexyl 2-hydroxybenzoate (commercially available as Homosalate^{™}), Ethylhexyl Methoxycinnamate (commercially available as NeoHelipan^{®} AV), 2-ethylhexyl 2-cyano-3,3-diphenylacrylate (OCR; commercially available as Octocrylene^{™}), 2-Hydroxy-4-methoxybenzophenone (commercially available as Oxybenzone^{™}), 2-ethyl-hexyl-4-methoxy cinnamate (MCX; commercially available as Parsol MCX^{™}) and mixtures thereof. Examples of water soluble UVB organic sunscreens include phenyl benzimidazole sulphonic acid.

Preferably, UVB organic sunscreens that may be used in the composition are selected from OCR, MCX and mixtures thereof.

When incorporated in the composition, UVB organic sunscreens may preferably be incorporated from 0.1 to 10 wt%, more preferably from 0.5 to 7 wt%, even more preferably from 1 to 5 wt%, further more preferably from 1 to 3.5 wt%, yet more preferably from 1 to 3 wt%, still more preferably 1 to 2.5 wt% in the composition.

Preferably, the composition further comprises one or more thickening agents. Examples of thickening agents that may be incorporated in the composition include, Acrylamide/Sodium Acryloyldimethyltaurate Copolymer (Aristoflex AVC), Hydroxyethyl Acrylate/Sodium Acryloyldimethyltaurate Copolymer, Aluminum Starch Octenyl Succinate, Polyacrylates (such as Carbomers including Carbopol^{®} 980, Carbopol^{®} 1342, Pemulen TR-2^{®} and the Ultrez^{®} thickeners), Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose).

Preferably, the composition comprises thickeners in an amount ranging from 0.05 to 10 wt%, more preferably from 0.3 to 2 wt%.

Preferably, the composition further comprises preservatives to protect against the growth of potentially harmful microorganisms. Examples of ingredients that may be used as preservatives in the composition include alkyl esters of para-hydroxybenzoic acid, hydantoin derivatives, propionate salts, and a variety of quaternary ammonium compounds. More preferably, ingredients that may be used as preservative in the composition are sodium benzoate, iodopropynyl butyl carbamate, methylisothiazolinone, iodopropynylbutylcarbamate, phenoxyethanol, methyl paraben, propyl paraben, imidazolidinyl urea, sodium dehydroacetate, ethylhexylglycerin, benzyl alcohol, alkane diols and mixtures thereof. The alkane diols that are suitable for use as preservative are C₆-C₁₂ alkanes that are vicinally substituted with hydroxy groups. Illustrative examples include 1,2-octane diol (caprylyl glycol), 2,3-octane diol, 1,2-nonane diol, 1,2-decane diol, 1,2-hexane diol, 3,4-octane diol, mixtures thereof or the like where caprylyl glycol is typically the most preferred.

When present in the composition, preservatives are added preferably in an amount 0.001 to 5 wt%, more preferably 0.01 to 3 wt% and most preferably 0.02 to 2 wt%.

Preferably, the composition further comprises vitamins and flavonoids. Examples of vitamins include Vitamin B₂, Vitamin C, ascorbyl phosphate and Biotin, Vitamin A (retinol), Vitamin A Palmitate, ascorbyl tetraisopalmitate, sodium ascorbyl phosphate , Vitamin E (tocopherol), Vitamin E Acetate and DL-panthenol. A particularly suitable Vitamin B₆ derivative is Pyridoxine Palmitate. Examples of preferred flavonoids include glucosyl hesperidin and rutin. Preferably, the composition comprises vitamins or flavonoids, collectively or individually in an amount from 0.001 to 10 wt%, more preferably from 0.01 to 5 wt%, and even more preferably from 0.1 to 3 wt%. Preferably, the composition further comprises herbal extracts. Examples of herbal extracts include pomegranate, white birch (Betula Alba), green tea, chamomile, licorice, boswellia serrata, olive (Olea Europaea) leaf, arnica montana flower, lavandula angustifolia, and extract combinations thereof. The extracts may either be water soluble or water-insoluble carried in a solvent which respectively is hydrophilic or hydrophobic. Water and ethanol are the preferred extract solvents.

Preferably, the composition further comprises a range of other optional ingredients that include binders, biological additives, buffering agents, colorants, astringents, fragrance, opacifying agents, conditioners, exfoliating agents, pH adjusters and skin healing agents.

The composition may be a leave-on composition or a wash-off composition. Preferably, the composition is a leave-on composition.

The following examples are provided to facilitate an understanding of the invention.

### Examples

### Example 1: Measurement of pickup

### Protocol

A group of expert panelist trained in assessing product sensorials e.g. product absorption, appearance, product pick up, ease of spread, ease of product absorption on a scale of 1 to 5 where a score equal to 1 being the most difficult and that equal to 5 being the most easy. The composition is detailed in Table 1. This composition was inside the scope of the present invention.

**Table 1**

| **Ingredient** | **Wt%** |
|---|---|
| Niacinamide | 3 |
| Glycerin | 5 |
| Mineral Oil | 7 |
| Petrolatum | 10 |
| Emulsifier | 2. |
| Titanium dioxide | 2 |
| Talc | 14 |
| Dow Corning@ MQ-1640 Flake Resin | 2 |
| Isopropyl Myristate | 4 |
| Polyethylene Wax | 6.5 |
| Hexyl Resorcinol | 0.25 |
| Water and other minors | to 100 |

### Example 2: Comparative composition A

A comparative composition, like that in Table 1 was prepared but with one change. This composition was outside the scope of the present invention.

**Table 2**

| **Ingredient** | **Wt%** |
|---|---|
| Niacinamide | 3 |
| Glycerin | 5 |
| Mineral Oil | 7 |
| Petrolatum | 10 |
| Emulsifier | 2 |
| Titanium dioxide | 2 |
| Talc | 0 |
| Dow Corning^{®} MQ-1640 Flake Resin | 2 |
| Isopropyl Myristate | 4 |
| Polyethylene Wax | 6.5 |
| Hexyl Resorcinol | 0.25 |
| Water and other minors | to 100 |

When the composition of Table 2 was observed, it was found that droplets of oils had separated from the main matrix thereby indicating that the composition was not stable.

### Example 3: Comparative composition B

Another comparative composition, like that in Table 3 was prepared but with one change. This composition was also outside the scope of the present invention.

**Table 3**

| **Ingredient** | **Wt%** |
|---|---|
| Niacinamide | 3 |
| Glycerin | 5 |
| Mineral Oil | 7 |
| Petrolatum | 10 |
| Emulsifier | 2 |
| Titanium dioxide | 2 |
| Talc | 14 |
| Dow Corning^{®} MQ-1640 Flake Resin | 2 |
| Isopropyl Myristate | 4 |
| Polyethylene Wax | 0 |
| Hexyl Resorcinol | 0.25 |
| Water and other minors | to 100 |

When the composition of Table 3 was observed, it was found that droplets of oils had separated from the main matrix thereby indicating that the composition was not stable.

## Claims

1. A cosmetic composition comprising:
a) from 0.01 to 20 wt% film-forming polymer having a contact angle of at least 85° where contact angle means the angle at which a water/vapor interface meets a solid surface at a temperature of 25°C which is measured with a goniometer or other water droplet shape analysis systems with water droplet of 5 µL and at 25°C,
b) from 0.01 to 20 wt% wax,
c) from 11 to 25 wt% inorganic structurant selected from the group consisting of talc, silica, mica, alumina, clays and mixtures thereof; and
d) from 5 to 50 wt% water, wherein the composition further comprises skin lightening agent selected from niacinamide, vitamin B6, 12-hydroxystearic acid, glutathione precursors, galardin, 4-alkyl substituted resorcinol and mixtures thereof, and wherein the wax is selected from a group consisting of beeswax, rice bran wax, montan wax, spermaceti wax, carnauba wax, candelilla wax, sugarcane wax, insect wax, hydrocarbon wax and mixtures thereof;
wherein the composition further comprises from 10 to 40 wt% emollient selected from the group consisting of mineral oil, petroleum jelly, isoparaffins, polyalphaolefins, isohexadecane and mixtures thereof

2. The composition according to claim 1 wherein the composition further comprises from 0.05 to 5 wt% optical particle selected from the group consisting of titanium dioxide, zinc oxide, zirconium oxide, iron oxide and mixtures thereof.

3. The composition according claims 1 or 2 wherein the film-forming polymer comprises a silicone resin.

4. The composition according to claim 3 wherein the silicone resin is MQ silicone resin, T silicone resin or a mixture thereof.

5. The composition according to claims 3 or 4 wherein the silicone resin is trimethylsiloxy silicate and/or polypropyl silsesquioxane.

6. The composition according to any one of claims 1 to 5 wherein the wax selected is hydrocarbon wax.

7. The composition according to any one of claims 1 to 6 wherein the composition comprises from 20 to 45 wt% water.

8. The composition according to any one of claims 1 to 79 wherein the inorganic structurant is talc.

9. The composition according to any one of claims 1 to 8 wherein the composition is in the form of a leave-on composition.

## Patentansprüche

1. Kosmetische Zusammensetzung, umfassend:
a) 0,01 bis 20 Gew.-% filmbildendes Polymer mit einem Kontaktwinkel von mindestens 85°, wobei der Kontaktwinkel den Winkel bedeutet, bei dem eine Wasser/Dampf-Grenzfläche bei einer Temperatur von 25°C auf eine feste Oberfläche trifft, gemessen mit einem Goniometer oder anderen Systemen zur Analyse der Wassertröpfchenform mit einem Wassertröpfchen von 5 µl und bei 25°C,
b) 0,01 bis 20 Gew.-% Wachs,
c) 11 bis 25 Gew.-% anorganisches Strukturierungsmittel, ausgewählt aus der Gruppe, bestehend aus Talk, Siliciumdioxid, Glimmer, Aluminiumoxid, Tonen und Mischungen davon; und
d) 5 bis 50 Gew.-% Wasser, wobei die Zusammensetzung außerdem ein hautaufhellendes Mittel umfasst, ausgewählt aus Niacinamid, Vitamin B6, 12-Hydroxystearinsäure, Glutathion-Vorläufern, Galardin, 4-Alkylsubstituiertem Resorcin und Mischungen davon, und wobei das Wachs aus einer Gruppe ausgewählt ist, bestehend aus Bienenwachs, Reiskleiewachs, Montanwachs, Walratwachs, Carnaubawachs, Candelillawachs, Zuckerrohrwachs, Insektenwachs, Kohlenwasserstoffwachs und Mischungen davon;
wobei die Zusammensetzung außerdem 10 bis 40 Gew.-% Weichmacher umfasst, ausgewählt aus der Gruppe, bestehend aus Mineralöl, Vaseline, Isoparaffinen, Polyalphaolefinen, Isohexadecan und Mischungen davon.

2. Zusammensetzung nach Anspruch 1, wobei die Zusammensetzung außerdem 0,05 bis 5 Gew.-% optische Partikel umfasst, ausgewählt aus der Gruppe, bestehend aus Titandioxid, Zinkoxid, Zirconiumoxid, Eisenoxid und Mischungen davon.

3. Zusammensetzung nach den Ansprüchen 1 oder 2, wobei das filmbildende Polymer ein Silikonharz umfasst.

4. Zusammensetzung nach Anspruch 3, wobei das Silikonharz MQ-Silikonharz, T-Silikonharz oder eine Mischung davon ist.

5. Zusammensetzung nach den Ansprüchen 3 oder 4, wobei das Silikonharz Trimethylsiloxysilikat und/oder Polypropylensilsesquioxan ist.

6. Zusammensetzung nach irgendeinem der Ansprüche 1 bis 5, wobei das ausgewählte Wachs ein Kohlenwasserstoffwachs ist.

7. Zusammensetzung nach irgendeinem der Ansprüche 1 bis 6, wobei die Zusammensetzung 20 bis 45 Gew.-% Wasser umfasst.

8. Zusammensetzung nach irgendeinem der Ansprüche 1 bis 7, wobei das anorganische Strukturierungsmittel Talk ist.

9. Zusammensetzung nach irgendeinem der Ansprüche 1 bis 8, wobei die Zusammensetzung in Form einer Leave-on-Zusammensetzung vorliegt.

## Revendications

1. Composition cosmétique comprenant :
a) de 0,01 à 20 % en masse de polymère formant un film ayant un angle de contact d'au moins 85° où l'angle de contact indique l'angle sous lequel une interface eau/vapeur rencontre une surface solide à une température de 25°C qui est mesuré avec un goniomètre ou d'autres systèmes d'analyse de forme de gouttelette d'eau avec une gouttelette d'eau de 5 µL et à 25°C,
b) de 0,01 à 20 % en masse de cire,
c) de 11 à 25 % en masse de structurant inorganique choisi dans le groupe consistant en stéatite, silice, mica, alumine, argiles et mélanges de ceux-ci ; et
d) de 5 à 50 % en masse d'eau, dans laquelle la composition comprend de plus un agent éclaircissant la peau choisi parmi niacinamide, vitamine B6, acide 12-hydroxystéarique, précurseurs de glutathione, galardine, résorcinol substitué par un 4-alkyle et mélanges de ceux-ci, et dans laquelle la cire est choisie dans un groupe consistant en cire d'abeille, cire d'écorce de riz, cire de lignite, cire de spermacéti, cire de carnauba, cire de candelilla, cire de sucre de canne, cire d'insecte, cire hydrocarbonée et mélanges de celles-ci ;
dans laquelle la composition comprend de plus de 10 à 40 % en masse d'émollient choisi dans le groupe consistant en huile minérale, gelée de pétrole, isoparaffines, polyoléfines alpha, isohexadécane et mélanges de ceux-ci.

2. Composition selon la revendication 1, dans laquelle la composition comprend de plus de 0,05 à 5 % en masse de particule optique choisie dans le groupe consistant en dioxyde de titane, oxyde de zinc, oxyde de zirconium, oxyde de fer et mélanges de ceux-ci.

3. Composition selon la revendication 1 ou 2, dans laquelle le polymère formant un film comprend une résine de silicone.

4. Composition selon la revendication 3, dans laquelle la résine de silicone est une résine de silicone MQ, résine de silicone T ou un mélange de celles-ci.

5. Composition selon la revendication 3 ou 4, dans laquelle la résine de silicone est un triméthylsiloxysilicate et/ou polypropylsilsesquioxane.

6. Composition selon l'une quelconque des revendications 1 à 5, dans laquelle la cire choisie est une cire hydrocarbonée.

7. Composition selon l'une quelconque des revendications 1 à 6, dans laquelle la composition comprend de 20 à 45 % en masse d'eau.

8. Composition selon l'une quelconque des revendications 1 à 79, dans laquelle le structurant inorganique est la stéatite.

9. Composition selon l'une quelconque des revendications 1 à 8, dans laquelle la composition est dans la forme d'une composition sans rinçage.
